# EUROPEAN PATENT SPECIFICATION

(11) **EP 4 076 233 B1**
(45) Date of publication and mention of the grant of the patent: **16.07.2025**
(21) Application number: 20838977.5
(22) Date of filing: 18.12.2020
(51) Int. Cl.: A61B 17/66, A61B 17/72

(54) **DISTRACTION DEVICE WITH REFLECTOR**
DISTRAKTIONSVORRICHTUNG MIT REFLEKTOR
DISPOSITIF DE DISTRACTION AVEC RÉFLECTEUR

(30) Priority: 20.12.2019 US 201916722141
(43) Date of publication of application: 26.10.2022
(73) Proprietor: NuVasive, Inc., San Diego, CA 92121 (US)
(72) Inventor: CHEVALIER, Éric, 91120 Palaiseau (FR); KERSPERN, Valentin, 75015 Paris (FR)
(74) Representative: Morabito, Sara
(86) International application number: PCT/EP2020/087261
(87) International publication number: WO 2021/123339

(56) References cited:
- EP-A1- 3 491 998
- EP-B1- 3 491 998
- WO-A2-2011/119873

## Description

### FIELD OF INVENTION

The present invention relates to the field of an implantable bone distraction device.

In particular, the present invention relates to a device for distracting osteotomically separated bone sections.

### BACKGROUND OF INVENTION

The distraction osteogenesis is a surgical procedure aiming at lengthening a bone. The lengthening occurs with the creation of new bone matter in the space between the two formerly linked bone segments. This new bone is called the callus and its quality and stiffness is a key parameter to sustain the mechanical constraints. The monitoring of the callus regeneration during the distraction phase of the procedure is of great interest to adjust the distraction osteogenesis protocol. Indeed, if the callus turns out to be too soft, the distraction step should be postponed to give additional time to the callus regeneration process. On the contrary, if the callus strengthens too early, the distraction length cannot be reached, and a new surgical intervention is required to resume the distraction. The monitoring of the callus is also advantageous once the expected length has been reached. Indeed, once the expected length is reached, the distraction is stopped and the callus is stabilized. The distraction device is then removed once the callus is consolidated and a rigid mechanical link is established between the bone segments.

The prior art includes the US patent US 5,364,396 which discloses an implantable bone distraction device and a method associated. This implantable bone device comprises two blocks, each fixed to a separated bone section and linked together to a rotatable drive rod and a drive rod actuator. The actuation of the drive rod actuator leads to a rotation of the rotatable drive rod actuator which move apart the two blocks.

However, in this device no means of monitoring bone reconstruction exists. In such conditions, the practitioner cannot determine whether the bone structure is regenerating properly.

Document WO 2011/119873A2 discloses a system according to the preamble of claim 1.

The review of the state of the art demonstrates that a need exists for an implantable system allowing the remote monitoring of bone tissue regeneration. This system would allow the patient and the caregivers to evaluate and monitor over time the local quality and the evolution of the bone tissue.

### SUMMARY

The present invention relates to a system for evaluating the evolution of the structure of a bone of a subject, said system comprising:
- an implantable medical device comprising a distraction body and at least one reflector coupled to the distraction body, said distraction being configured to distract osteotomically separated bone section bodies and comprising:
- a first block for implantation and attachment to a first bone section,
- a second block for implantation and attachment to a second bone section separated from the first bone section by an osteotomy,
- an actuator configured to adjust the space between the first block and the second block when activated, enabling distraction between the first bone section and the second bone section,
wherein said at least one reflector being configured to reflect an electromagnetic signal and being embedded in a surrounding tissue of the subject when the distraction body is attached to the bone of the subject,
- a calculation module configured to compute a parameter representative of the structure of the bone of the subject surrounding the bone distraction device, said parameter being computed from a reflected signal corresponding to a reflection, on the reflector embedded in the surrounding tissue of the subject, of an excitation signal comprising at least one frequency in the characteristic frequency range of the reflector, said reflected signal being representative of a dielectric constant of the surrounding tissue.

In one embodiment, the system further comprises:
- an emitting module configured to emit the excitation signal comprising at least one frequency in the characteristic frequency range of the reflector;
- a receiving module configured to receive the reflected signal corresponding to a reflection of the excitation signal emitted by the emitting module on the reflector embedded in the surrounding tissue of the subject.

In one embodiment, at least two modules among the emitting module, the receiving module and the calculation module are integrated in a same external non-invasive device. This embodiment allows to have an external non-invasive device more convenient and functional.

In one embodiment, the reflector is configured to reflect the excitation signal applied to the reflector.

In one embodiment, the reflector has a plane shape or a bent shape. The different reflector shapes allow to adapt the reflector to the surface of the different implant bodies.

In one embodiment, the system comprises at least two reflectors arranged at different positions relative to the implant body. Several reflectors allow to have an accurate measure of the bone regeneration. Several reflectors on the implant body allow to monitor local information regarding to the bone quality.

In one embodiment, the calculation module is configured to compute a geometric mapping of the parameter representative of the structure of the bone of the subject from the reflected signal associated to different reflectors and their respective positions relative to the distraction body.

In one embodiment, the parameter representative of the structure of the bone of the subject is computed from a comparison between the reflected signal and a model establishing a correlation between, on one hand, a reflected signal on said reflector and its surrounding tissue and, on the other hand, said parameter representative of the structure of the bone.

In one embodiment, the parameter representative of the structure of the bone of the subject is computed from a comparison between the reflected signal and a reflected signal obtained previously. The comparison allows to determine the status of the bone regeneration.

In one embodiment, the reflector is fixed to the distraction body either to the first block, the second block or the actuator.

In one embodiment, the implantable bone distraction device according, further comprises a drive rod having a first end received in a first chamber bore defined by the first block and a second end, opposite to said first end, received in a second chamber bore defined by the second block, the drive rod being able to adjust the space between the first block and the second block, wherein the first chamber bore is a drive chamber bore whereas the second chamber bore is a threaded bore, the second end of the drive rod being a threaded end threadably received in the threaded bore of the second block, wherein the actuator cooperates with the drive rod to rotate the drive rod, said drive rod being able to adjust the space between the first block and the second block.

Also described herein although not falling within the scope of the claims is a method for evaluating the evolution of the structure of a bone of a subject using the system according to the invention with an implant body intended to be attached to the bone of the subject and at least one reflector coupled to the implant body, said reflector being configured to reflect an electromagnetic signal and being in contact with the surrounding tissue of the subject when the implant body is attached to the bone of the subject, said method comprising steps of:
- emitting an excitation signal comprising at least one frequency in the characteristic frequency range of the reflector;
- receiving a reflected signal corresponding to a reflection of the excitation signal emitted by the emitting module on the reflector embedded in the surrounding tissue of the subject, said reflected signal being representative of a dielectric constant of the surrounding tissue;
- computing, from the received reflected signal, a parameter representative of the structure of the bone of the subject.

### DEFINITIONS

In the present invention, the following terms have the following meanings:
- **"Bone regeneration"** refers to the physiological of bone segments welding. The bone at the interface progressively gets denser and stiffer up to creating a rigid mechanical link between the two adjacent bone segments.
- **"reflector"** refers to a component being configured to reflect an electromagnetic signal, and being embedded in a surrounding tissue of the subject when an implant body is attached to the bone of the subject.

### BRIEF DESCRIPTION OF THE DRAWINGS

The following detailed description will be better understood when read in conjunction with the drawings. For the purpose of illustrating, the system is shown in the preferred embodiments. It should be understood, however that the application is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

Features and advantages of the invention will become apparent from the following description of embodiments of a system, this description being given merely by way of example and with reference to the appended drawings in which:
**Figure 1a** is a first perspective view of a medical device implant with a reflector,
**Figure 1b** is a second perspective view of another medical device implant with a reflector,
**Figure 2a** is a first perspective view of a medical device implant with screwed ends and a longitudinal reflector,
**Figure 2b** is a second perspective view of another medical device implant with screwed ends and a reflector wrapped around the implant,
**Figure 3a** is a first positioning of a medical device implant with a reflector inside a bone,
**Figure 3b** is a second positioning of a medical device implant with a reflector on a bone surface.

### DETAILED DESCRIPTION

While various embodiments have been described and illustrated, the detailed description is not to be construed as being limited hereto. Various modifications can be made to the embodiments by those skilled in the art without departing from the scope of the disclosure as defined by the claims.

The first aspect of the present invention relates to a system for evaluating the evolution of the structure of a bone of a subject during a bone distraction comprising at least one reflector to monitor the bone healing process between the two separated bone sections and a calculation module.

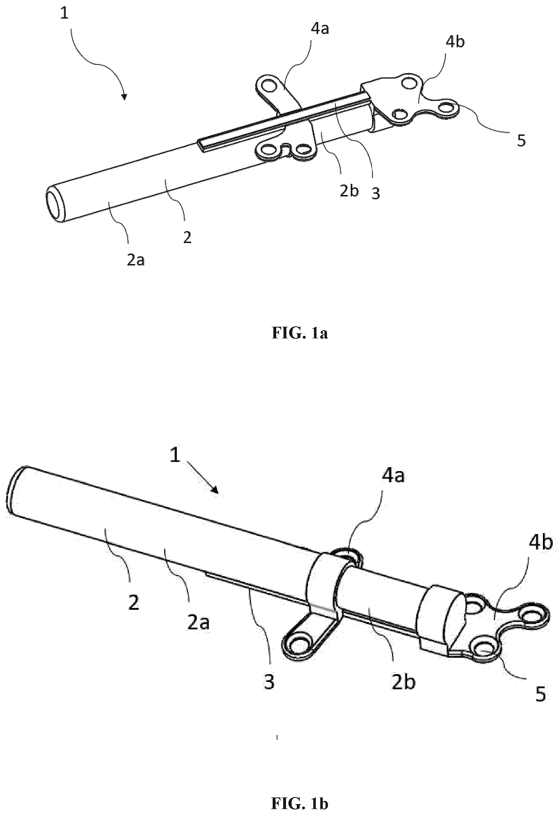

As shown in Figures 1a and 1b, a system for evaluating the evolution of the bone structure of a subject is presented. The system comprises an implantable medical device 1, said implantable medical device 1 comprising an implant body 2 and a reflector 3. In these embodiments, the implant body 2 is a distraction device implanted on the two separated bone sections. The implantable medical device 1 can be implanted on any bone permitting distraction or into the bone to distract. The implant body 2 may be made of various materials such as metal, polymer or ceramic for example. In another embodiment, the implant body 2 also may be made of carbon graphite fiber.

As illustrated in Fig. 1a, the implantable medical device 1 comprises a first block 2a for implantation and attachment to a first bone section, said first block 2a defining a first chamber bore, located in the inner part of the implantable medical device 1 (not represented). The implantable medical device 1 comprises a second block 2b for implantation and attachment to a second bone section separated from the first bone section by an osteotomy, said second block defining a second chamber bore. In one embodiment, the first block 2a is configured to receive the second block 2b.

The second block 2b is able to slide inside the first block 2a, permitting to adjust the space between the first block 2a and the second block 2b. The first and second blocks 2a and 2b are cylindrical but in another embodiment, the first and the second blocks 2a and 2b may have another shape such as a rectangle. The dimensions of the implantable medical device 1 are defined according to the dimensions of the distracted bone. The implantable medical device 1 is made so as to fit to the distracted bone.

The implantable medical device 1 further comprises an actuator to adjust the space between the first block 2a and second block 2b when activated, enabling distraction between the first bone section and second bone section. Different types of actuators can be used in the system according to the invention. One possibility would be a motor with a worm screw, or even a magnetic coupling system as known by the man skilled in the art. The inventors do not want to be limited to a specific actuator; it may be positioned in different places according to the needs.

The implantable medical device 1 further comprises a hook device divided in two parts 4a and 4b. The first hook device 4a is fixed on the first block 2a and the second hook device 4b is fixed on the second block 2b. The first hook device 4a comprises three arms with at least one bore on each end of each arm. The number of arms is not limited. The first hook device 4a turns around the first part of the implant body 2. The second hook device 4b is fixed at the end of the second block 2b. The second hook device 4b is cap-shaped but may have another shape in another embodiment. The second hook device 4b has three arms with at least one bore 5 on each end of each arm. The number of arms is not limited.

The at least one bore 5 is configured to receive at least one screw for screwing the implant body on the bone surface or inside the bone.

The implantable medical device in the figures 1a and 1b, further comprises at least one reflector 3 able to be arranged in several positions on the implant body 2 for monitoring the regeneration of the bone between the first block 2a and second block 2b during a distraction. The reflector 3 is in contact with a surrounding tissue of the subject when the implant body 2 is positioned. The at least one reflector 3 is configured to reflect an electromagnetic signal in a characteristic frequency ranging between 1 MHz and 50GHz. Preferably, the characteristic frequency ranges between 500 MHz and 15 GHz. The reflector 3 may amplify and/or filter the reflected signal for improving the signal-noise-ratio. The number of reflectors 3 on the implant body 2 is not limited.

In figures 1a and 1b, a reflector is positioned on the first hook device 4a of the implant body 2. In other embodiments, the reflector may be placed in another position such as on the first block 2a or the second block 2b of the implant body 2 or each part of the implant body 2. The number of reflectors on the implant body 2 is not limited. In this embodiment, the reflectors 3 have a rectangular shape. In other embodiments, the reflectors 3 may have another shape such as a circular or a square shape. The several reflectors 3 may have different shapes for providing specific and identifiable reflected signals. The relative positions of each identifiable reflector 3 may be known at the implantation step.

In another embodiment, the at least one reflector 3 is in part the implant body 2 itself. For example, the first block 2a or the second block 2b of the implant body 2 may be configured to emit an electromagnetic wave. In another embodiment, the implant body 2 may be the reflector 3 itself. Each part of the implant body 2 may reflect an electromagnetic wave.

In another embodiment, the implant body 2 may be the reflector itself comprising at least one hole with a complementary shape of a reflector 3 permitting to reflect an electromagnetic signal without a reflector. In this embodiment, the implant body may be printed in 3D for example and may be made of titanium.

In another embodiment, the reflector 3 may be a passive implantable reflector and, in specific embodiments, the reflector 3 may be a resonator. For example, the resonator may be a split ring resonator or a dipole antenna.

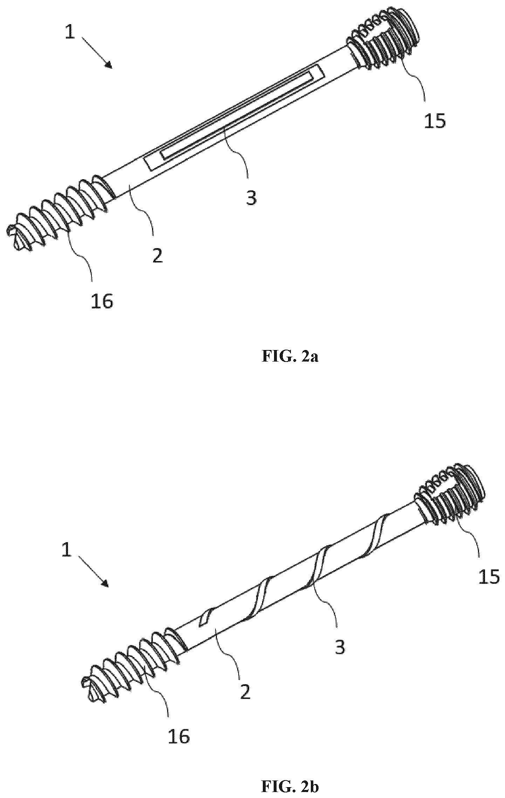

As shown in figures 2a and 2b, screws for fixing the implant body 2 can be associated to the implantable medical device 1. Said device 1, in figure 2a comprises a reflector 3. The implant bodies 2 may be made of various materials such as metal or polymer or ceramic for example. In another embodiment, the implant bodies 2 may be made of carbon graphite fiber.

In the figures 2a and 2b, the implant body 2 is a fixation screw. The screw passes through the bores 5 of the first and second hook device 4a and 4b for fixing the implant medical device 1 of the figures 1a and 1b to the bone. The implant body 2 of the figures 2a and 2b goes through the whole bone section and provides information of the progress of the bone regeneration.

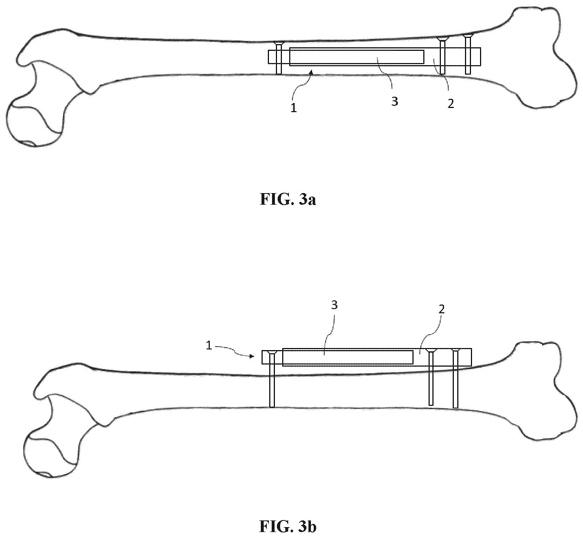

In the figures 2a and 2b, the implant body 2 has threaded ends, allowing to insert the threaded part into the bone by screwing it directly. In this embodiment, the implant body 2 is screwed by the front end 16 of the implant body 2. The rear end 15 of the implant body 2 is threaded following the movement of the front end 16 and allowing the implantation of the implantable medical device 1. The reflector 3 of figure 2a extends longitudinally along the surface of the bone, while the reflector of figure 2b is wrapped around the surface of the bone in a threaded like configuration. The rear end 15 has a larger diameter than the front end 16 for locking the first part of the implant body on the bone. The rear end 15 is outside the sectioned bone, see figure 3b for this embodiment. The rear end 15 is locked into one of the bores of the first and second hook devices 4a and 4b of the implant body in this embodiment. In another embodiment, the rear end 15 is inside the sectioned bone, see figure 3a for this embodiment, when the implant body 2 is fixed directly into the bone to distract.

According to one preferred embodiment, the system for evaluating the evolution of a bone further comprises a calculation module (not represented in the figures) configured to compute a parameter representative of the structure of the bone of the subject.

The emission module emits an excitation signal comprising at least one frequency, the reflector 3 receives the excitation signal in the surrounding tissue of the subject and reflects a signal to the calculation module.

The reflected signal leads to a parameter representative of the structure of the bone. After the measurement, the calculation module compares said parameter to a previous measurement. This comparison leads to an indicator of the evolution of the structure of the bone. In another embodiment, the calculation module compares said parameter to a model or a predefined threshold to determine the indicator of the progress of the bone regeneration.

In an embodiment, the calculation module is a noninvasive device. In another embodiment, the calculation module may be an invasive device combined with the implantable medical device 1 for example. The parameter is computed from a reflected signal corresponding to a reflection on the reflector 3 in contact with the surrounding tissue, of an excitation signal. The reflected signal is representative of at least one electrical property such as a dielectric constant of the surrounding tissue.

The dielectric parameters of the bone tissue correlated well with its composition. For example, relative permittivity at frequencies between 100 kHz and 5 MHz are different than tissue with a sparse structure. Microstructural parameters related to the surface of trabecular structure were found to be the main determinants of relative permittivity. This suggests that the variation in different microstructural elements may be detected by various electrical parameters.

When an Electromagnetic wave is incident on human tissues some of the energy is transmitted and some is reflected back, because of impedance mismatches. The reflection and transmission coefficients vary from tissue to tissue and they are dependent on permittivity, conductivity, conductivity and frequency.

A reflected electromagnetic wave is then dependent of dielectric properties of tissues; the frequency analysis of reflected electromagnetic waves leads to the detection of tissue differentiation or contrast.

When a bone tissue regeneration occurs, there is an evolution of dielectric properties which can be monitored by reflected wave analysis (in frequency or in time).

Example: Osteoporosis is a disease evolving into a severe health condition the main symptom of which is a decrease in density and a violation of the bone tissue structure. The bone tissue generally consists of two layers: the outer layer is the cortical bone and the inner layer is the trabecular bone. When the bone tissue is damaged by osteoporosis, calcium is washed away from the trabecular bone. As a consequence, the gradually formed pores and cracks are filled with liquid components: fat and bone marrow. As a result, the development of osteoporosis leads to a change in the physical characteristics of the trabecular bone, in particular, the complex dielectric constant.

According to another embodiment, the system for evaluating the evolution of a bone comprises an emitting module configured to emit the excitation signal, the excitation signal comprising at least one frequency in the characteristic frequency range of the reflector 3.

The system also comprises a receiving module configured to receive a reflected signal; the reflected signal corresponding to a reflection of the excitation signal emitted by the emitting module on the reflector 3 in contact with the surrounding tissue of the subject. The emitting and receiving modules may be only one noninvasive device. In another embodiment, the emitting module, the receiving module and the calculation module are coupled in one noninvasive device.

In an embodiment, the non-invasive device is configured to individually identify the reflected signal of each reflector 3 of the implantable medical device. In another embodiment, the non-invasive device is configured to display the parameter of the progress of the bone regeneration. The parameter is computed for each reflector 3 of the implantable medical device. The advantage of this embodiment is that the practitioner may make his diagnosis based on the measurement provided by a specific reflector 3 located according to the information required. In another embodiment, the parameter may be computed based on a synthesis of the different reflected signals of the several reflectors 3. The advantage of this embodiment is that the practitioner may make his diagnosis based on the set of measurements provided by each reflector 3 which represents a global information of the regeneration bone progress.

In another embodiment, the non-invasive device is configured to display the estimated time remaining before the total regeneration and formation of the bone between the first block 2a and the second block 2b. With such information, the subject may adapt his daily life activities accordingly.

After the implantation of the implantable medical device 1 on the bone or inside the bone, a process of bone regeneration is expected between the first block 2a and the second block 2b after a distraction step. The progress of the bone regeneration process is able to modify the excitation signal during the reflection of the reflector 3.

The implantable medical device is exposed to the excitation when the practitioner uses the non-invasive device. The measurement may be made every month for example. In another embodiment, the measurement is continuous. The frequency of the measurement is not limited.

## Claims

1. A system for evaluating the evolution of the structure of a bone of a subject, said system comprising:
- an implantable medical device (1) comprising a distraction body (2) and at least one reflector (3) coupled to the distraction body (2), said distraction body (2) being configured to distract osteotomically separated bone section bodies and comprising:
- a first block (2a) for implantation and attachment to a first bone section,
- a second block (2b) for implantation and attachment to a second bone section separated from the first bone section by an osteotomy,
- an actuator configured to adjust the space between the first block (2a) and the second block (2b) when activated, enabling distraction between the first bone section and the second bone section,
wherein said at least one reflector (3) is configured to reflect an electromagnetic signal and is embedded in a surrounding tissue of the subject when the distraction body (2) is attached to the bone of the subject,
- a calculation module configured to compute a parameter representative of the structure of the bone of the subject surrounding the bone distraction device, **characterised in that**
said
parameter is computed from a reflected signal corresponding to a reflection, on the reflector (3) embedded in the surrounding tissue of the subject, of an excitation signal comprising at least one frequency in the characteristic frequency range of the reflector (3), said reflected signal being representative of a dielectric constant of the surrounding tissue.

2. The system according to claim 1, further comprising:
- an emitting module configured to emit the excitation signal comprising at least one frequency in the characteristic frequency range of the reflector (3);
- a receiving module configured to receive the reflected signal corresponding to a reflection of the excitation signal emitted by the emitting module on the reflector (3) embedded in the surrounding tissue of the subject.

3. The system according to claim 2, wherein at least two modules among the emitting module, the receiving module and the calculation module are integrated in one external non-invasive device.

4. The system according to any one of claims 1 to 3, wherein the reflector (3) has a plane shape.

5. The system according to any one of claims 1 to 3, wherein the reflector (3) has a bent shape.

6. The system according to any one of claims 1 to 5, wherein the calculation module is configured to compute a geometric mapping of the parameter representative of the structure of the bone of the subject from the reflected signal associated to different reflectors (3) and their respective positions relative to the distraction body.

7. The system according to any one of claims 1 to 6, wherein the parameter representative of the structure of the bone of the subject is computed from a comparison between the reflected signal and a model establishing a correlation between, on one hand, a reflected signal on said reflector (3) and its surrounding tissue and, on the other hand, said parameter representative of the structure of the bone.

8. The system according to any one of claims 1 to 7, wherein the parameter representative of the structure of the bone of the subject is computed from a comparison between the reflected signal and a reflected signal obtained previously.

9. The system according to any one of claims 1 to 8, wherein the reflector (3) is fixed to the distraction body (2) either to the first block, the second block (2b) or the actuator.

10. The system according to any of claims 1 to 9, further comprising a drive rod having a first end received in a first chamber bore defined by the first block (2a) and a second end, opposite to said first end, received in a second chamber bore defined by the second block (2b), the drive rod being able to adjust the space between the first block (2a) and the second block (2b), wherein the first chamber bore is a drive chamber bore and the second chamber bore is a threaded bore, the second end of the drive rod being a threaded end threadably received in the threaded bore of the second block (2b), and wherein
the actuator cooperates with the drive rod to rotate the drive rod, said drive rod being able to adjust the space between the first block (2a) and the second block (2b).

## Patentansprüche

1. System zur Bewertung der Entwicklung der Struktur eines Knochens eines Subjekts, wobei das System aufweist:
- eine implantierbare medizinische Vorrichtung (1), die einen Distraktionskörper (2) und zumindest einen mit dem Distraktionskörper (2) verbundenen Reflektor (3) aufweist, wobei der Distraktionskörper (2) eingerichtet ist, um osteotomisch getrennte Knochenabschnittskörper zu distrahieren, und aufweist:
- einen ersten Block (2a) zur Implantation und Befestigung an einem ersten Knochenabschnitt,
- einen zweiten Block (2b) zur Implantation und Befestigung an einem zweiten Knochenabschnitt, der durch eine Osteotomie vom ersten Knochenabschnitt getrennt ist,
- einen Aktuator, der eingerichtet ist, um bei Aktivierung den Abstand zwischen dem ersten Block (2a) und dem zweiten Block (2b) einzustellen, wobet eine Distraktion zwischen dem ersten Knochenabschnitt und zweiten Knochenabschnitt ermöglicht wird,
- wobei zumindest ein Reflektor (3) eingerichtet ist, um ein elektromagnetisches Signal zu reflektieren und in ein umgebendes Gewebe des Subjekts eingebettet ist, wenn der Distraktionskörper (2) am Knochen des Subjekts befestigt ist,
- ein Berechnungsmodul, das eingerichtet ist, um einen Parameter zu berechnen, der für die Struktur des Knochens des Subjekts um die Knochendistraktionsvorrichtung repräsentativ ist, **dadurch gekennzeichnet, dass** der Parameter aus einem reflektierten Signal berechnet wird, das einer Reflexion eines Anregungssignals mit zumindest einer Frequenz im charakteristischen Frequenzbereich des Reflektors (3) am im umgebenden Gewebe des Subjekts eingebetteten Reflektor (3) entspricht, wobei das reflektierte Signal für eine Dielektrizitätskonstante des umgebenden Gewebes repräsentativ ist.

2. System nach Anspruch 1, ferner umfassend:
- ein Sendemodul, das eingerichtet ist, um das Anregungssignal auszusenden, das zumindest eine Frequenz im charakteristischen Frequenzbereich des Reflektors (3) aufweist;
- ein Empfangsmodul, das eingerichtet ist, um das reflektierte Signal zu empfangen, das einer Reflexion des vom Sendemodul ausgesendeten Anregungssignals am Reflektor (3) entspricht, der im umgebenden Gewebe des Subjekts eingebettet ist.

3. System nach Anspruch 2, wobei zumindest zwei Module zwischen dem Sendemodul, dem Empfangsmodul und dem Berechnungsmodul in einer externen nicht-invasiven Vorrichtung integriert sind.

4. System nach einem der Ansprüche 1 bis 3, wobei der Reflektor (3) eine ebene Form aufweist.

5. System nach einem der Ansprüche 1 bis 3, wobei der Reflektor (3) eine gebogene Form aufweist.

6. System nach einem der Ansprüche 1 bis 5, wobei das Berechnungsmodul eingerichtet ist, um eine geometrische Abbildung des für die Struktur des Knochens des Subjekts repräsentativen Parameters aus dem reflektierten Signal zu berechnen, das verschiedenen Reflektoren (3) und ihren jeweiligen Positionen relativ zum Distraktionskörper zugeordnet ist.

7. System nach einem der Ansprüche 1 bis 6, wobei der Parameter, der für die Struktur des Knochens des Subjekts repräsentativ ist, aus einem Vergleich zwischen dem reflektierten Signal und einem Modell berechnet wird, das einerseits eine Korrelation zwischen einem reflektierten Signal am Reflektor (3) und seinem umgebenden Gewebe und andererseits dem Parameter, der für die Struktur des Knochens repräsentativ ist, herstellt.

8. System nach einem der Ansprüche 1 bis 7, wobei der Parameter, der für die Struktur des Knochens des Subjekts repräsentativ ist, aus einem Vergleich zwischen dem reflektierten Signal und einem zuvor erhaltenen reflektierten Signal berechnet wird.

9. System nach einem der Ansprüche 1 bis 8, wobei der Reflektor (3) am Distraktionskörper (2) entweder am ersten Block, am zweiten Block (2b) oder am Aktuator befestigt ist.

10. System nach einem der Ansprüche 1 bis 9, das ferner eine Antriebsstange mit einem ersten Ende, das in einer ersten Kammerbohrung aufgenommen ist, die durch den ersten Block (2a) gebildet wird, und mit einem zweiten Ende aufweist, das dem ersten Ende gegenüberliegt und in einer zweiten Kammerbohrung aufgenommen ist, die durch den zweiten Block (2b) gebildet wird, wobei die Antriebsstange in der Lage ist, um den Abstand zwischen dem ersten Block (2a) und zweiten Block (2b) einzustellen, wobei die erste Kammerbohrung eine Antriebskammerbohrung und die zweite Kammerbohrung eine Gewindebohrung ist und das zweite Ende der Antriebsstange ein Gewindeende ist, das schraubbar in der Gewindebohrung des zweiten Blocks (2b) aufgenommen ist, und wobei der Aktuator mit der Antriebsstange zusammenwirkt, um die Antriebsstange zu drehen, wobei die Antriebsstange in der Lage ist, um den Abstand zwischen dem ersten Block (2a) und zweiten Block (2b) einzustellen.

## Revendications

1. Système d'évaluation de l'évolution de la structure d'un os d'un sujet, ce système comprenant :
- un dispositif médical implantable (1) comprenant un corps de distraction (2) et au moins un réflecteur (3) couplé au corps de distraction (2), ledit corps de distraction (2) étant configuré pour distraire des corps de section osseuse séparés par ostéotomie et comprenant :
- un premier bloc (2a) pour l'implantation et la fixation à une première section osseuse,
- un deuxième bloc (2b) destiné à être implanté et fixé à une deuxième section osseuse séparée de la première section osseuse par une ostéotomie,
- un actionneur configuré pour ajuster l'espace entre le premier bloc (2a) et le deuxième bloc (2b) lorsqu'il est activé, permettant la distraction entre la première section osseuse et la deuxième section osseuse,
dans lequel ledit au moins un réflecteur (3) est configuré pour réfléchir un signal électromagnétique et est intégré dans un tissu environnant du sujet lorsque le corps de distraction (2) est fixé à l'os du sujet,
- un module de calcul configuré pour calculer un paramètre représentatif de la structure de l'os du sujet entourant le dispositif de distraction osseuse,
**caractérisé en ce que**
ledit paramètre est calculé à partir d'un signal réfléchi correspondant à une réflexion, sur le réflecteur (3) noyé dans le tissu environnant du sujet, d'un signal d'excitation comprenant au moins une fréquence dans la gamme de fréquences caractéristiques du réflecteur (3), ledit signal réfléchi étant représentatif d'une constante diélectrique du tissu environnant.

2. Système selon la revendication 1, comprenant en outre :
- un module d'émission configuré pour émettre le signal d'excitation comprenant au moins une fréquence dans la gamme de fréquences caractéristiques du réflecteur (3) ;
- un module de réception configuré pour recevoir le signal réfléchi correspondant à une réflexion du signal d'excitation émis par le module d'émission sur le réflecteur (3) noyé dans le tissu environnant du sujet.

3. Système selon la revendication 2, dans lequel au moins deux modules parmi le module d'émission, le module de réception et le module de calcul sont intégrés dans un dispositif externe non invasif.

4. Système selon l'une des revendications 1 à 3, dans lequel le réflecteur (3) a une forme plane.

5. Système selon l'une des revendications 1 à 3, dans lequel le réflecteur (3) a une forme courbée.

6. Système selon l'une des revendications 1 à 5, dans lequel le module de calcul est configuré pour calculer une cartographie géométrique du paramètre représentatif de la structure de l'os du sujet à partir du signal réfléchi associé à différents réflecteurs (3) et à leurs positions respectives par rapport au corps de distraction.

7. Système selon l'une quelconque des revendications 1 à 6, dans lequel le paramètre représentatif de la structure de l'os du sujet est calculé à partir d'une comparaison entre le signal réfléchi et un modèle établissant une corrélation entre, d'une part, un signal réfléchi sur ledit réflecteur (3) et son tissu environnant et, d'autre part, ledit paramètre représentatif de la structure de l'os.

8. Système selon l'une des revendications 1 à 7, dans lequel le paramètre représentatif de la structure de l'os du sujet est calculé à partir d'une comparaison entre le signal réfléchi et un signal réfléchi obtenu précédemment.

9. Système selon l'une des revendications 1 à 8, dans lequel le réflecteur (3) est fixé au corps de distraction (2) soit au premier bloc, soit au deuxième bloc (2b), soit à l'actionneur.

10. Système selon l'une des revendications 1 à 9, comprenant en outre une tige d'entraînement ayant une première extrémité reçue dans un premier alésage de chambre défini par le premier bloc (2a) et une deuxième extrémité, opposée à ladite première extrémité, reçue dans un deuxième alésage de chambre défini par le deuxième bloc (2b), la tige d'entraînement peut ajuster l'espace entre le premier bloc (2a) et le deuxième bloc (2b), dans lequel le premier alésage de chambre est un alésage de chambre d'entraînement et le deuxième alésage de chambre est un alésage fileté, la deuxième extrémité de la tige d'entraînement étant une extrémité filetée reçue de manière filetée dans l'alésage fileté du deuxième bloc (2b), et dans lequel
l'actionneur coopère avec la tige d'entraînement pour faire tourner la tige d'entraînement, ladite tige d'entraînement pouvant ajuster l'espace entre le premier bloc (2a) et le deuxième bloc (2b).
